# EUROPEAN PATENT SPECIFICATION

(11) **EP 0 346 881 B1**
(45) Date of publication and mention of the grant of the patent: **01.09.1993**
(21) Application number: 89110820.1
(22) Date of filing: 14.06.1989
(51) Int. Cl.: C07D 307/42, C07B 35/06, C07B 41/02, C07B 49/00

(54) **A process for producing furylpropargylcarbinol and a derivate thereof**
Verfahren zur Herstellung von Furylpropargylcarbinol und einem Derivat davon
Procédé de préparation de furylpropargylcarbinol et un dérivé de celui-ci

(30) Priority: 16.06.1988 JP 149829/88; 05.07.1988 JP 167922/88; 28.10.1988 JP 273910/88; 31.10.1988 JP 276849/88; 17.11.1988 JP 291906/88; 07.03.1989 JP 55784/89
(43) Date of publication of application: 20.12.1989
(73) Proprietor: SUMITOMO CHEMICAL COMPANY, LIMITED, Chuo-ku Osaka 541 (JP)
(72) Inventor: Saito, Kenji, Hirakata-shi (JP); Matsuo, Sanshiro, Sakai-shi (JP); Oda, Yoshiaki, Toyonaka-shi (JP)
(74) Representative: Henkel, Feiler, Hänzel & Partner

(56) References cited:
- EP-A- 0 113 107
- EP-A- 0 247 589
- PATENT ABSTRACTS OF JAPAN vol. 12, no. 242 (C-510)(3089), 8 July 1988.
- PATENT ABSTRACTS OF JAPAN vol 12, no. 248 (C-511)(3095), 13 July 1988
- JOURNAL OF ORGANIC CHEMISTRY vol. 50, no. 6, 22 March 1985, pages 910-912; C PETRIER et al.:"Allylzine Reagent Additions in Aqueous Media"
- HOUBEN-WEYL: "METHODEN DER ORGANISCHEN CHEMIE" vol. V/2A, Georg Thieme Verlag Stuttgart, 1977, pages 78-104

## Description

The present invention relates to a process for producing furylpropargylcarbinol or a derivative thereof represented by the formula (I): 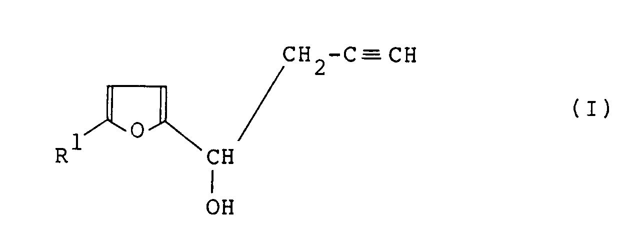
wherein R¹ represents a hydrogen atom or a methyl group.

The furylpropargylcarbinol and a derivative thereof are very important as intermediates of agricultural chemicals, perfumes and medicines, and particularly applicable to an intermediate of prallethrin.

As a process for producing furylpropargylcarbinol or a derivative thereof, there has heretofore been known a process using the Grignard reaction of propargyl bromide or propargyl chloride with a furfural-type compound (Japanese Patent Application Kokai No. 59-118780).

Since, however, propargyl bromide and propargyl chloride are detonable or capable of monopropellant-type burning, in view of safety, the inhibition of the detonability is required in the industrial bulk use of them. Therefore, the above-mentioned process is not always an industrially advantageous one.

The present inventors have found a process for producing furylpropargylcarbinol or a derivative thereof represented by the formula (I), using neither propargyl bromide nor propargyl chloride.

The present invention relates to a process for producing furylpropargylcarbinol or a derivative thereof represented by the formula (I) which comprises subjecting a haloallylfurancarbinol represented by the formula (II): 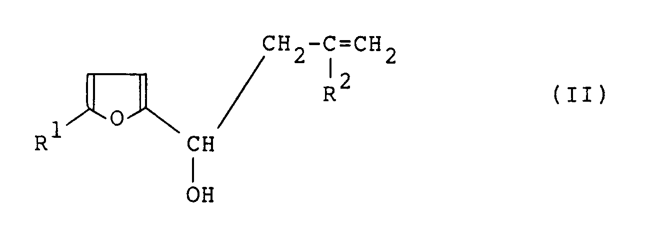
wherein R¹ represents a hydrogen atom or a methyl group and R² represents a chlorine atom, a bromine atom or an iodine atom, to a dehydrohalogenation reaction using a base in a solvent.

An object of the present invention is to provide a process for producing furylpropargylcarbinol and derivatives thereof being important as intermediates of agricultural chemicals, prefumes and medicines, and particularly applicable as intermediates of prallethrin.

Another object of the present invention is to provide a novel haloallylfurancarbinol or a derivative thereof represented by the formula (II).

Other objects and advantages will become apparent from the following description.

The base used in the dehydrohalogenation reaction of the present invention includes organolithium compounds such as n-butyllithium, sec-butyllithium, tert-butyllithium, benzyllithium, allyllithium, vinyllithium, phenyllithium, methyllithium and the like; alkali metal amides such as sodium amide, lithium amide, lithium diisopropylamide, lithium dicyclohexylamide, lithium bis(trimethylsilyl)amide and the like; alkali metals such as lithium, sodium, potassium and the like; alkali metal alcoholates such as sodium methoxide, potassium tert-butoxide and the like; alkali metal hydroxides and alkaline earth metal hydroxides such as lithium hydroxide, sodium hydroxide, potassium hydroxide, magnesium hydroxide, calcium hydroxide, barium hydroxide and the like; and alkali metal carbonates and alkaline earth metal carbonates such as lithium carbonate, sodium carbonate, potassium carbonate, magnesium carbonate, calcium carbonate, barium carbonate, lithium hydrogencarbonate, sodium hydrogencarbonate, potassium hydrogencarbonate, magnesium hydrogencarbonate, calcium hydrogencarbonate, barium hydrogencarbonate and the like.

The used amount of the base is usually 1-15 parts by mole, preferably 1-10 parts by mole for 1 part by mole of haloallylfurancarbinol.

The type of the reaction solvent depends upon the type of the base used.

When an organolithium compound or an alkali metal amide is used as the base, there is used as the reaction solvent, alone or in admixture, a hydrophilic aprotic solvent inert to the dehydrohalogenation reaction such as tetrahydrofuran, dioxane or the like, a hydrophobic solvent inert to the dehydrogenation such as n-pentane, n-hexane, n-heptane, benzene, toluene, xylene or the like.

When an alkali metal is used as the base, liquid ammonia is used as the reaction solvent.

When an alkali metal alcoholate is used as the base, there is used as the reaction solvent, alone or in admixture, a hydrophobic or hydrophilic ether such as ethyl ether, isopropyl ether, tetrahydrofuran, dioxane, diglyme, triglyme or the like; a hydrophobic hydrocarbon such as n-pentane, n-hexane, n-heptane, benzene, toluene, xylene or the like; an alcohol such as methanol, ethanol, propanol, butanol or the like; a hydrophobic halogenated hydrocarbon such as carbon tetrachloride, chloroform, methylene dichloride, chlorobenzene, dichlorobenzene or the like; an amine such as pyridine, triethylamine or the like; and a polar aprotic solvent such as acetonitrile, dimethylsulfoxide, N,N-dimethylformamide, N-methylpyrrolidone or the like. Further, as far as a solvent has no influence on the dehydrohalogenation reaction, there may be used alone or in admixture a ketone such as acetone, methyl ethyl ketone, methyl isobutyl ketone or the like.

When a hydroxide or a carbonate of any of an alkali metal or an alkaline earth metal is used as the base, there is used, as the reaction solvent, the one exemplified above in the case of using the alkali metal alcoholate as the base, water, and a mixture of water and said reaction solvent exemplified above. There is preferably used a mixture of water and a hydrophobic hydrocarbon such as toluene, xylene, pentane, hexane or the like or a mixture of water and a hydrophobic halogenated hydrocarbon such as monochlorobenzene, dichlorobenzene or the like.

The above-mentioned solvent can be used alone or in admixture, and the used amount thereof is not particularly critical. The used amount is usually 1-20 parts by weight for 1 part by weight of the used haloallylfurancarbinol or the derivative thereof (II). The used amount of water is 1-4 parts by weight for 1 part by weight of the used hydroxide or carbonate of any of an alkali metal and an alkaline earth metal.

A reaction temperature is usually selected from a range of from -70°C to 120°C depending upon the combination of a base and a solvent.

Reaction time is not particularly critical. The end point of the reaction can be considered to be a point of time when the haloallylfurancarbinol or the derivative thereof (II) used as a raw material is entirely expended.

To the reaction system, if necessary, can be added a phase transfer catalyst. The used amount thereof is usually 0.01-5 parts by mole for 1 part by mole of the used haloallylfurancarbinol or the derivative thereof (II).

The phase transfer catalyst includes organic quarternary ammonium salts such as tetra-n-butylammonium bromide, tetra-n-butylammonium chloride, tetra-n-pentylammonium bromide, tetra-n-pentylammonium iodide, benzyltriethylammonium chloride, benzyltriethylammonium bromide, benzyltripropylammonium chloride, benzyltripropylammonium iodide, cetyltrimethylammonium chloride and the like; organic quaternary phosphonium salts such as tetraphenylphosphonium chloride, tetraphenylphosphonium bromide, tetraphenylphosphonium iodide, benzyltriphenylphosphonium chloride and the like; macrocyclic ethers such as 18-crown-6, 15-crown-5, 12-crown-4 and the like.

Preferably, the alkali metal hydroxide or the alkaline earth metal hydroxide is used as the base. More preferably is used the alkali metal hydroxide.

Preferably, the polar aprotic solvent is used as the reaction solvent. Particularly when the phase transfer catalyst is used, there are preferably used water alone or a mixture of water and any of the hydrophobic hydrocarbon and the hydrophobic halogenated hydrocarbon, too.

After the dehydrohalogenation reaction is completed, the furylpropargylcarbinol or the derivative thereof (I) can be obtained from the reaction mixture by, for example, the pouring of water, extraction, separation and the like followed by the distillation of an organic phase.

The haloallylfurancarbinol or the derivative thereof (II) as a raw material of the process of the present invention can be produced by reacting, in the presence of zinc in a solvent, furfural or a derivative thereof represented by the formula (III): 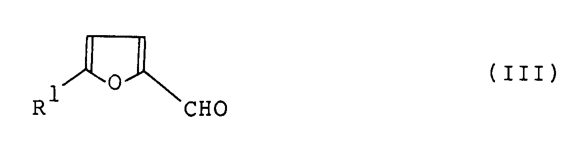
wherein R¹ represents a hydrogen atom or a methyl group, with an organic dihalide compound represented by the formula (IV): 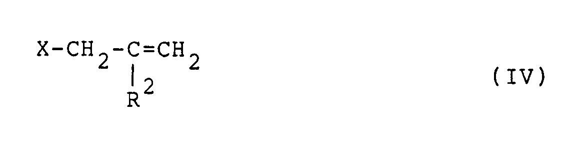
wherein each of X and R² individually represents a chlorine atom, a bromine atom or an iodine atom. Hereinafter, the reaction of furfural or a derivative thereof (III) with an organic dihalide compound (IV) is referred to as the haloallylfurancarbinol-producing reaction.

The organic dihalide compound (IV) includes 2,3-dichloro-1-propene, 2,3-dibromo-1-propene, 2,3-diiodo-1-propene, 2-chloro-3-bromo-1-propene, 2-chloro-3-iodo-1-ropene, 2-bromo-3-chloro-1-propene, 2-bromo-3-iodo-1-propene, 2-iodo-3-chloro-1-propene, 2-iodo-3-bromo-1-propene and the like.

The used amount of the organic dihalide compound (IV) is usually 1-3 parts by mole for 1 part by mole of furfural or a derivative thereof (III).

Zinc having various shapes on the market can be used. Zinc powder or zinc particles are preferably used. More preferably, zinc powder is used.

The used amount of zinc is 0.8-3 parts by weight, preferably 1.1-1.5 parts by weight for 1 part by weight of furfural or a derivative thereof (III).

There are used, if necessary, halogenated ammoniums such as ammonium chloride, ammonium bromide, ammonium iodide and the like. The used amount thereof is 0.15-6 parts by weight for 1 part by weight of furfural or a derivative thereof (III).

As the solvent of the haloallylfurancarbinol-producing reaction, there is used water alone or a mixture of water and an organic solvent. The organic solvent includes tetrahydrofuran, dioxane, toluene, benzene, monochlorobenzene, ethylene dichloride, a polar aprotic solvent such as acetonitrile, diglyme, dimethylsulfoxide, N,N-dimethylformamide, N-methylpyrrolidone, etc., and the like.

When the solvent is a mixture, the solvent should contain water as a main component. The content of water in the mixture is preferably 50% by weight or more.

The amount of water in the solvent is usually 3-24 parts by weight, preferably 4-19 parts by weight for 1 part by weight of furfural or a derivative thereof (III).

To the reaction of the haloallylfurancarbinol-producing reaction, if necessary, can be added a phase transfer catalyst. The used amount thereof is usually 0.05-0.4 parts by weight for 1 part by weight of furfural or a derivative thereof (III).

The phase transfer catalyst includes the above-mentioned organic quaternary ammonium salts.

A reaction temperature of the haloallylfurancarbinol-producing reaction is in a range of 0°C-100°C, preferably 15°C-50°C.

Reaction time of the haloallylfurancarbinol-producing reaction is usually 1-8 hours and, however, it can be shortened by the addition of a small amount of an acid.

The acid includes acetic acid, hydrochloric acid, sulfuric acid and the like.

When acetic acid is used as the acid, the added amount thereof is preferably 5% by weight or less based on the water contained in the solvent. When hydrochloric acid or sulfuric acid is used as the acid, the added amount thereof is preferably 0.1% by weight based on the water contained in the solvent.

After the haloallylfurancarbinol-producing reaction is completed, the haloallylfurancarbinol (II) can be obtained from the reaction mixture by, for example, filtration, separation followed by the distillation of an organic phase.

Thus, according to the process of the present invention, furylpropargylcarbinol and derivatives thereof represented by the formula (I) can be produced in safety and industrially with advance.

The present invention will be explained more specifically below referring to Examples.

### REFERENTIAL EXAMPLE 1

Into a reactor were charged 380 g of 30%-aqueous sodium hydroxide and 2.4 g of 68%-aqueous solution of benzyltriethylammonium chloride and the mixture was stirred at 20-30°C for 30 minutes. The mixture was cooled down to 10°C, and thereinto, 340 g of 30%-aqueous sodium hydroxide and 340 g of crude 1,2,3-trichloropropane having a purity of 73% were dropped together at 10-15°C in 25 minutes. The mixture was stirred at 10-15°C for 90 minutes, and thereafter, 300 g of water was added thereto. The resulting mixture was subjected to separation to obtain 248 g of an organic phase.

The content of 2,3-dichloro-1-propene in the organic phase was 71.7% by weight (yield: 95.2%). the content of 1,2,3-trichloropropane was 0.2% by weight or less.

The organic phase was subjected to simple distillation to obtain 170 g of 2,3-dichloro-1-propene having a purity of 93.8%.

### EXAMPLE 1

(1) Into a reactor were charged 20.0 g of 5-methylfurfural, 88 g of water, 33 g of toluene and 26 g of zinc powder and the mixture was kept at 33-35°C. Thereinto, 44.4 g of distilled 2,3-dichloro-1-propene obtained in Referential Example 1 was dropped in 20 minutes. The resulting mixture was kept at 33-35°C for 4 hours. After the reaction was completed, the crystals derived from the zinc powder were filtered off. To the filtrate was added 66 g of toluene and a toluene phase was separated. The toluene phase was washed with 30 g of 7%-aqueous sodium carbonate and subsequently with 50 g of water, and thereafter, toluene was distilled off at 60°C or less. The resulting residue was subjected to simple distillation to obtain 30.5 g of 2′-chloroallyl-5-methylfurylcarbinol.
   Boiling point: 84-85°C/0.8 mmHg.
   Yield: 90%.
   NMR data (measurement solvent: CDCl₃, internal standard: TMS, chemical shift: δ-value): 2.27 (s, 3H), 2.75-2.92 (m, 2H), 4.95 (dd, 1H), and 5.26 (s, 2H), 5.89 (d, 1H) and 6.13 (d, 1H).
(2) The mixture of 12.3 g of 2′-chloroallyl-5-methylfurylcarbinol obtained in (1), 42.3 g of 50%-aqueous sodium hydroxide, 37 g of toluene and 15.1 g of benzyltriethylammonium chloride was stirred at 40°C for 8 hours. To the mixture were added 100 g of toluene and 100 g of water, and extraction and separation were carried out. An organic phase was washed with 5%-aqueous hydrochloric acid and subsequently with 7%-aqueous sodium carbonate, and thereafter, the organic phase was concentrated under a reduced pressure.

The resulting residue was subjected to simple distillation to obtain 7.86 g of 5-methylfurylpropargylcabinol.

Boiling point: 74°C/0.7 mmHg.

Yield: 79.2%.

NMR data (measurement solvent: CDCl₃, internal standard: TMS, chemical shift: δ-value): 2.03 (t, 1H, J=2.6Hz), 2.25 (d, 3H, J=1.0Hz), 2.70 (dd, 2H, J=6.6Hz, 2.6Hz), 2.89 (brs, 1H), 4.76 (t, 1H, J=6.6Hz), 5.88 (dq, 1H, J=3.3Hz, 10Hz) and 6.17 (d, 1H, J=3.3Hz).

### EXAMPLE 2

The same reaction, after-treatment and purification were repeated as in Example 1, except that 17.5 g of furfural was substituted for 20.0 g of 5-methylfurfural. As a result, there were obtained 2-chloroallylfurylcarbinol as an intermediate and furylpropargylcarbinol as the last product.

### EXAMPLE 3

The mixture of 12.3 g of 2′-chloroallyl-5-methylfurylcarbinol, 42.3 g of 50%-aqueous sodium hydroxide, 37 g of toluene and 21.3 g of tetra-n-butylammonium bromide was stirred at 40°C for 8 hours. Thereafter, the same procedure was repeated as in Example 1 (2) to obtain 7.94 g of 5-methylfurylpropargylcarbinol (yield: 80.0%).

### EXAMPLE 4

The mixture of 20.0 g of 2-chloroallylfurylcarbinol, 97.4 g of 40%-aqueous potassium hydroxide, 40 g of hexane and 28.1 g of trioctylmethylammonium bromide was stirred at room temperature for 24 hours. Thereafter, the same procedure was repeated as in Example 1 (2) to obtain 13.0 g of furylpropargylcarbinol (yield: 82.3%).

### EXAMPLE 5

The mixture of 10.0 g of 2′-chloroallyl-5-methylfurylcarbinol, 42.9 g of 50%-aqueous sodium hydroxide and 10 g of toluene was stirred at 80°C for 5 hours. An organic phase was separated and washed with water, and thereafter, concentrated under a reduced pressure.

The resulting residue was subjected to simple distillation to obtain 5-methylfurylpropargylcarbinol.

### EXAMPLE 6

Into the mixture of 22.0 g of 5-methylfurfural, 265 g of 12.5%-aqueous ammonium chloride, 2.6 g of tetrabutylammonium bromide, 33 g of toluene and 26 g of zinc powder, 44.4 g of 2,3-dichloro-1-propene was dropped at 33-35°C in an hour. The resulting mixture was stirred at the same temperature for 3 hours. After the reaction was completed, the crystals derived from the zinc powder was filtered off and 66 g of toluene was added to the filtrate. A toluene phase was separated and washed with 60 g of 15%-aqueous sodium hydrogensulfite and subsequently with 50 g of water, and thereafter, toluene was distilled off at 60°C or less. The resulting concentrated residue was subjected to simple distillation to obtain 32.0 g of 2′-chloroallyl-5-methylfurylcarbinol.

Boiling point: 84-85°C/0.8 mmHg.

Yield: 85%.

18.6 g of the resulting 2′-chloroallyl-5-methylfurylcarbinol was dissolved in 100 ml of methyl ethyl ketone, and thereto was added 276 g of potassium carbonate. The mixture was heated up to 80°C and kept at the same temperature for 62 hours. After the mixture was cooled, insoluble matter was filtered off. The filtrate was washed with saturated brine and dried with zeolite. The drying agent was filtered off and the filtrate was concentrated under a reduced pressure. Thereafter, the purification by distillation was carried out to obtain 5-methylfurylpropargylcarbinol.

### EXAMPLE 7

10.0 g of 2′-chloroallyl-5-methylfurylcarbinol was dissolved in 100 g of dimethylformamide. To the solution was added 6.43 g of sodium hydroxide. The resulting mixture was kept at 40°C for 3 hours. The mixture was neutralized with 10%-aqueous hydrochloric acid, and thereafter, the solvent was distilled off under a reduced pressure. The resulting residue was added to a mixture of toluene and water. An organic phase was separated and thereafter concentrated under a reduced pressure. Thereafter, the organic phase was purified by distillation to obtain 6.92 g of 5-methylfurylpropargylcarbinol (yield: 86.0%).

### EXAMPLE 8

The mixture of 18 g of furfural, 33 g of toluene, 88 g of 3%-aqueous acetic acid and 26 g of zinc powder was kept at 33-35°C with stirring.

44.4 g of 2,3-dichloro-1-propene was dropped thereinto at the same temperature in 20 minutes, and thereafter, the resulting mixture was kept at the same temperature for 2 hours. After the reaction was completed, the crystals derived from zinc powder was filtered off. To the filtrate was added 66 g of toluene, and a toluene phase was separated. The toluene phase was washed with 30 g of 7%-aqueous sodium carbonate and subsequently with 50 g of water. Thereafter, toluene was distilled off at 60°C or less. The resulting residue was subjected to simple distillation to obtain 29.4 g of 2-chloroallylfurylcarbinol.

Boiling point: 74°C/0.7 mmHg.

Yield: 91.0%.

The same dehydrohalogenation reaction and after-treatment were repeated as in Example 4 using the resulting 2-chloroallylfurylcarbinol, except that 40%-aqueous sodium hydroxide was substituted for 40%-aqueous potassium hydroxide. As a result, furylpropargylcarbinol was obtained.

### EXAMPLE 9

Into the mixture of 11.0 g of 5-methylfurfural, 38.4 g of 3%-aqueous acetic acid, 43.2 g of toluene and 13.1 g of zinc powder, 40.0 g of 2,3-dibromo-1-propene was dropped at 30-35°C in 20 minutes. After the dropping was completed, the resulting mixture was stirred at 30-35°C for 2 hours. After the reaction was completed, the crystals derived from the zinc powder were filtered off and the obtained crystals were washed with 86 g of toluene. The filtrate and the washed liquid were combined to obtain a filtrate mixture. A toluene phase was separated from the filtrate mixture. The toluene phase was washed with 30 g of 7%-aqueous sodium carbonate and subsequently with 50 g of water. Thereafter, toluene was distilled off at 60°C or less. The resulting residue was purified by column chromatography using 100 g of silica gel as a packing and ethyl acetate as an eluent. Thereby, 11.1 g of 2′-bromoallyl-5-methylfurylcarbinol was obtained.

Yield: 48.2%.

FD-Mass spectrum data: M⁺ 230, M⁺+2 232.

NMR data (measurement solvent: CDCl₃, internal standard: TMS, chemical shift: δ-value): 2.08 (brs, 1H), 2.28 (d, 3H, J=1.0Hz), 2.93 (m, 2H), 4.98 (dd,1H, J=5.1Hz, 8.4Hz), 5.54 (d, 1H, J=1.7Hz), 5.72 (d, 1H, J=1.7Hz), 5.91 (m, 1H), 6.16 (d, 1H, J=3.3Hz).

IR data: 3380 cm⁻¹ (O-H, stretching vibration), 1620 cm⁻¹ (vinylidene C=C, stretching vibration).

The same dehydrohalogenation reaction and after-treatment were repeated as in Example 1 (2), except that the obtained 2′-bromoallyl-5-methylfurylcarbinol was substituted for 2′-chloroallyl-5-methylfurylcarbinol. As a result, 5-methylfurylpropargylcarbinol was obtained.

### EXAMPLE 10

Into the mixture of 9.6 g of furfural, 38.4 g of 3%-aqueous acetic acid, 43.2 g of toluene and 13.1 g of zinc powder, 40.0 g of 2,3-dibromo-1-propene was dropped at 30-35°C in 20 minutes. After the dropping was completed, the resulting mixture was stirred at 30-35°C for 2 hours. After the reaction was completed, the crystals derived from zinc powder were filtered off and the obtained crystals were washed with 86 g of toluene. The filtrate and the wash liquid were combined to obtain a filtrate mixture. A toluene phase was separated from the filtrate mixture. The toluene phase was washed with 30 g of 7%-aqueous sodium carbonate and subsequently with 50 g of water. Toluene was distilled off at 60°C or less. The resulting residue was purified by column chromatography using 100 g of silica gel as a packing and ethyl acetate as an eluent. Thereby, 10.2 g of 2-bromoallylfurylcarbinol was obtained.

Yield: 47.2%.

FD-Mass spectrum data: M⁺ 216, M⁺+2 218.

NMR data (measurement solvent: CDCl₃, internal standard: TMS, chemical shift: δ-value): 2.28 (brs, 1H), 2.94 (m, 2H), 5.04 (dd, 1H, J=5.6Hz, 7.9Hz), 5.54 (d, 1H, J=1.7Hz), 5.71 (d, 1H, J=1.7Hz), 6.29 (m, 1H), 6.33 (m, 1H), 7.38 (m, 1H).

IR data: 3380 cm⁻¹ (O-H, stretching vibration), 1630 cm⁻¹ (vinylidene C=C, stretching vibration).

The same dehydrohalogenation reaction and after-treatment were repeated as in Example 8, except that the obtained 2-bromoallylfurylcarbinol was substituted for 2-chloroallylfurylcarbinol. As a result, furylpropargylcarbinol was obtained.

### EXAMPLE 11

Into the mixture of 11.0 g of 5-methylfurfural and 40.0 g of water was added each of 22.1 g of 2,3-dichloro-1-propene, 2.3 g of 50%-aqueous acetic acid and 13.1 g of zinc powder at 30-35°C in 3 hours. After the addition was completed, the resulting mixture was stirred at 30-35°C for 3 hours. After the reaction was completed, the crystals derived from the zinc powder were filtered off and the obtained crystals were washed with 120 g of toluene. The filtrate and the wash liquid were combined to obtain a filtrate mixture. A toluene phase was separated from the filtrate mixture. The toluene phase was washed with 30 g of 7%-aqueous sodium carbonate and subsequently with 50 g of water. Toluene was distilled off at 60°C or less. The resulting residue was subjected to simple distillation to obtain 13.2 g of 2′-chloroallyl-5-methylfurylcarbinol (yield: 70.5%).

The same dehydrohalogenation reaction and after-treatment were repeated as in Example 1 (2), using the obtained 2′-chloroallyl-5-methylfurylcarbinol. As a result, 5-methylfurylpropargylcarbinol was obtained.

## Claims

1. A process for producing furylpropargylcarbinol or a derivative thereof represented by the formula (I): 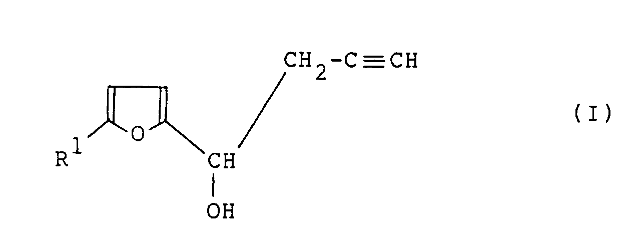 wherein R¹ represents a hydrogen atom or a methyl group, which comprises subjecting a haloallylfurancarbinol or a derivative thereof represented by the formula (II): 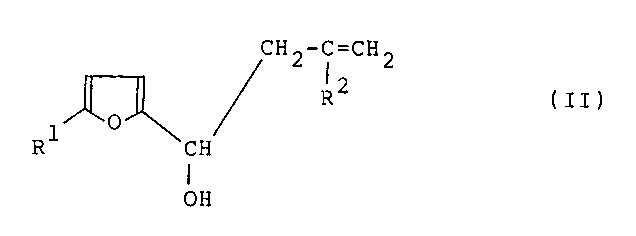 wherein R¹ represents a hydrogen atom or a methyl group and R² represents a chlorine atom, a bromine atom or an iodine atom, to a dehydrohalogenation reaction with a base in a reaction solvent.

2. A process for producing furylpropargylcarbinol or a derivative thereof according to Claim 1, wherein the base is an alkali metal hydroxide or an alkaline earth metal hydroxide.

3. A process for producing furylpropargylcarbinol or a derivative thereof according to Claim 1, wherein the reaction solvent is a mixture of water and any of a hydrophobic hydrocarbon or a hydrophobic halogenated hydrocarbon.

4. A process for producing furylpropargylcarbinol or a derivative thereof according to Claim 1, wherein the reaction solvent is water.

5. A process for producing furylpropargylcarbinol or a derivative thereof according to Claim 1, wherein the reaction solvent is a polar aprotic solvent.

6. A process for producing furylpropargylcarbinol or a derivative thereof according to Claim 2, wherein the reaction solvent is a mixture of water and any of a hydrophobinc hydrocarbon or a hydrophobic halogenated hydrocarbon.

7. A process for producing furylpropargylcarbinol or a dirivative thereof according to Claim 2, wherein the reaction solvent is water.

8. A process for producing furylpropargylcarbinol or a derivative thereof according to Claim 2, wherein the reaction solvent is a polar aprotic solvent.

9. A process for producing furylpropargylcarbinol or a derivative thereof according to Claim 1, wherein the dehydrohalogenation reaction is carried out in the presence of a phase transfer catalyst.

10. A process for producing furylpropargylcarbinol or a derivative thereof according to Claim 9, wherein the reaction solvent is water.

11. A process for producing furylpropargylcarbinol or a derivative thereof according to Claim 9, wherein the reaction solvent is a mixture of water and any of a hydrophobic hydrocarbon or a hydrophobic halogenated hydrocarbon.

12. A process for producing furylpropargylcarbinol or a derivative thereof according to Claim 2, wherein the dehydrohalogenation reaction is carried out in the presence of a phase transfer catalyst.

13. A process for producing furylpropargylcarbinol or a derivative thereof according to Claim 1, wherein the haloallylfurancarbinol or the derivative thereof is produced according to a process which comprises reacting furfural or a derivative thereof represented by the formula (III): 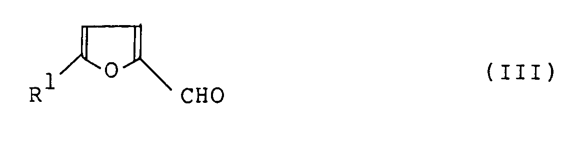 wherein R¹ represents a hydrogen atom or a methyl group, with an organic dihalide compound represented by the formula (IV): 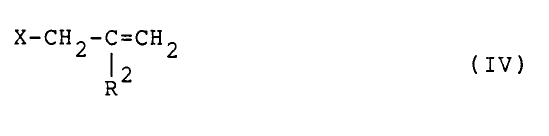 wherein each of R² and X independently represents a chlorine atom, a bromine atom or an iodine atom, in the presence of zinc in a solvent selected from water and a mixture of water and an organic solvent.

14. A process for producing furylpropargylcarbinol or a derivative thereof according to Claim 13, wherein the mixture of water and an organic solvent contains water as a major component.

15. A process for producing furylpropargylcarbinol or a derivative thereof according to Claim 13, wherein the reaction of furfural or a derivative thereof with an organic dihalide compound is carried out in the presence of an acid.

16. A process for producing furylpropargylcarbinol or a derivative thereof according to Claim 14, wherein the reaction of furfural or a derivative thereof with an organic dihalide compound is carried out in the presence of an acid.

17. A process for producing furylpropargylcarbinol or a derivative thereof according to Claim 15, wherein the acid is selected from the group consisting of acetic acid, hydrochloric acid and sulfuric acid.

18. A process for producing furylpropargylcarbinol or a derivative thereof according to Claim 13, wherein the organic dihalide compound is 2,3-dichloro-1-propene.

19. A process for producing furylpropargylcarbinol or a derivative thereof according to Claim 14, wherein the organic dihalide compound is 2,3-dichloro-1-propene.

20. A process for producing furylpropargylcarbinol or a derivative thereof according to Claim 15, wherein the organic dihalide compound is 2,3-dichloro-1-propene.

21. A process for producing furylpropargylcarbinol or a derivative thereof according to Claim 16, wherein the organic dihalide compound is 2,3-dichloro-1-propene.

22. A process for producing furylpropargylcarbinol or a derivative thereof according to Claim 17, wherein the organic dihalide compound is 2,3-dichloro-1-propene.

23. A process for producing furylpropargylcarbinol or a derivative thereof according to Claim 13, wherein the haloallylfurancarbinol is 2′-chloroallyl-5-methylfurylcarbinol or 2-chloroallylfurylcarbinol.

24. A process for producing furylpropargylcarbinol or a derivative thereof according to Claim 13, wherein the organic dihalide compound is 2,3-dibromo-1-propene.

25. A process for producing furylpropargylcarbinol or a derivative thereof according to any of Claims 18 and 24, wherein the base is an alkali metal hydroxide or an alkaline earth metal hydroxide.

26. A process for producing furylpropargylcarbinol or a derivative thereof according to Claim 25, wherein the solvent is a mixture of water and a polar aprotic solvent in the process which comprises reacting furfural or a derivative thereof represented by the formula (III) with an organic dihalide compound represented by the formula (IV).

27. A process for producing furylpropargylcarbinol or a derivative thereof according to Claim 25, wherein the solvent is water in the process which comprises reacting furfural or a derivative thereof represented by the formula (III) with an organic dihalide compound represented by the formula (IV).

28. A process for producing furylpropargylcarbinol or a derivative thereof according to Claim 25, wherein the solvent is a mixture of water and any of a hydrophobic hydrocarbon or a hydrophobic halogenated hydrocarbon.

29. A haloallylfurancarbinol or a derivative thereof represented by the formula (II): 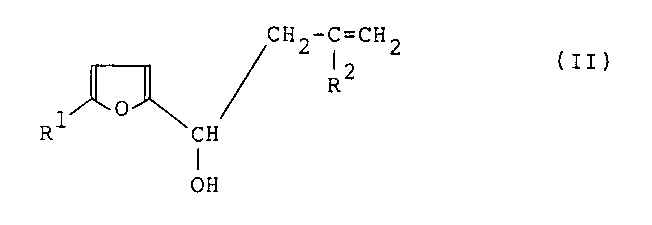 wherein R¹ represents a hydrogen atom or a methyl group and R² represents a chlorine atom, a bromine atom or an iodine atom.

30. A haloallylfurancarbinol or a derivative thereof according to Claim 29, wherein R¹ is a hydrogen atom and R² is a chlorine atom.

31. A haloallylfurancarbinol or a derivative thereof according to Claim 29, wherein R¹ is a methyl group and R² is a chlorine atom.

32. A haloallylfurancarbinol or a derivative thereof according to Claim 29, wherein R¹ is a hydrogen atom and R² is a bromine atom.

33. A haloallylfurancarbinol or a derivative thereof according to Claim 29, wherein R¹ is a methyl group and R² is a bromine atom.

34. A process for producing a haloallylfurancarbinol or a derivative thereof represented by the formula (II): 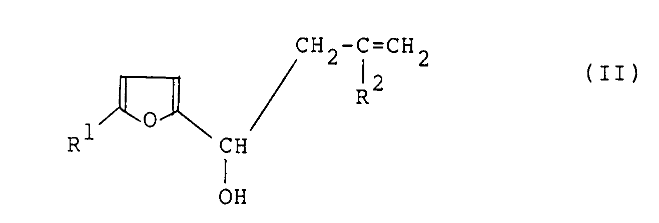 wherein R¹ represents a hydrogen atom or a methyl group and R² represents a chlorine atom, a bromine atom or an iodine atom, which comprises reacting furfural or a derivative thereof represented by the formula (III): 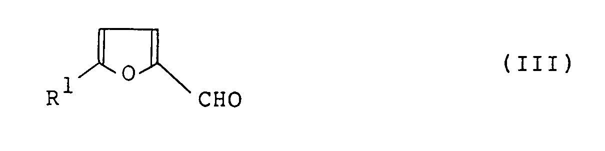 wherein R¹ represents a hydrogen atom or a methyl group, with an organic dihalide compound represented by the formula (IV): 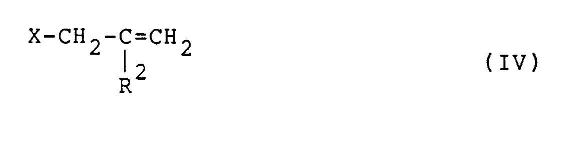 wherein each of R² and X independently represents a chlorine atom, a bromine atom or an iodine atom, in the presence of zinc in a solvent selected from water and a mixture of water and an organic solvent.

35. A process for producing a haloallylfurancarbinol or a derivative thereof according to Claim 34, wherein the organic dihalide compound is 2,3-dichloro-1-propene or 2,3-dibromo-1-propene.

36. A process for producing a haloallylfurancarbinol or a derivative thereof according to Claim 34, wherein the reaction is carried out in the presence of an acid.

37. A process for producing a haloallylfurancarbinol or a derivative thereof according to Claim 36, wherein the organic dihalide compound is 2,3-dichloro-1-propene or 2,3-dibromo-1-propene.

## Patentansprüche

1. Verfahren zur Herstellung von Furylpropargylcarbinol oder eines Derivats desselben der Formel (I) 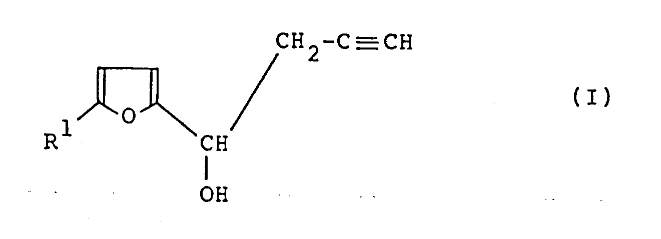 worin R₁ für ein Wasserstoffatom oder eine Methylgruppe steht, durch Enthydrohalogenieren eines Halogenallylfurancarbinols oder eines Derivat desselben der Formel (II), 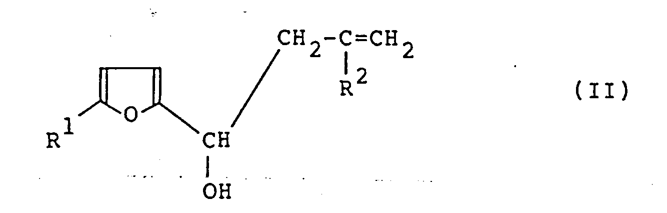 worin R₁ für ein Wasserstoffatom oder eine Methylgruppe steht und R₂ ein Chlor-, Brom- oder Jodatom darstellt, mit einer Base in einem Reaktionslösungsmittel.

2. Verfahren zur Herstellung von Furylpropargylcarbinol oder eines Derivats desselben nach Anspruch 1, wobei die Base aus einem Alkalimetallhydroxid oder einem Erdalkalimetallhydroxid besteht.

3. Verfahren zur Herstellung von Furylpropargylcarbinol oder eines Derivats desselben nach Anspruch 1, wobei das Reaktionslösungsmittel aus einem Gemisch aus Wasser und irgendeinem hydrophoben Kohlenwasserstoff oder hydrophoben halogenierten Kohlenwasserstoff besteht.

4. Verfahren zur Herstellung von Furylpropargylcarbinol oder eines Derivats desselben nach Anspruch 1, wobei das Rekationslösungsmittel aus Wasser besteht.

5. Verfahren zur Herstellung von Furylpropargylcarbinol oder eines Derivats desselben nach Anspruch 1, wobei das Reaktionslösungsmittel aus einem polaren aprotischen Lösungsmittel besteht.

6. Verfahren zur Herstellung von Furylpropargylcarbinol oder eines Derivats desselben nach Anspruch 2, wobei das Reaktionslösungsmittel aus einem Gemisch aus Wasser und irgendeinem hydrophoben Kohlenwasserstoff oder hydrophoben halogenierten Kohlenwasserstoff besteht.

7. Verfahren zur Herstellung von Furylpropargylcarbinol oder eines Derivats desselben nach Anspruch 2, wobei das Reaktionlösungsmittel aus Wasser besteht.

8. Verfahren zur Herstellung von Furylpropargylcarbinol oder eines Derivats desselben nach Anspruch 2, wobei das Reaktionslösungsmittel aus einem polaren aprotischen Lösungsmittel besteht.

9. Verfahren zur Herstellung von Furylpropargylcarbinol oder eines Derivats desselben nach Anspruch 1, wobei die Enthydrohalogenierungsreaktion in Gegenwart eines Phasentransferkatalysators stattfindet.

10. Verfahren zur Herstellung von Furylpropargylcarbinol oder eines Derivats desselben nach Anspruch 9, wobei das Reaktionslösungsmittel aus Wasser besteht.

11. Verfahren zur Herstellung von Furylpropargvlcarbinol oder eines Derivats desselben nach Anspruch 9, wobei das Reaktionslösungsmittel aus einem Gemisch aus Wasser und irgendeinem hydrophoben Kohlenwasserstoff oder hydrophoben halogenierten Kohlenwasserstoff besteht.

12. Verfahren zur Herstellung von Furylpropargylcarbinol oder eines Derivats desselben nach Anspruch 2, wobei die Enthydrohalogenierungsreaktion in Gegenwart eines Phasentransferkatalysators durchgeführt wird.

13. Verfahren zur Herstellung von Furylpropargylcarbinol oder eines Derivats desselben nach Anspruch 1, wobei das Halogenallylfurancarbinol oder dessen Derivat nach einem Verfahren, umfassend die Umsetzung von Furfural oder eines Derivats desselben der Formel (III), 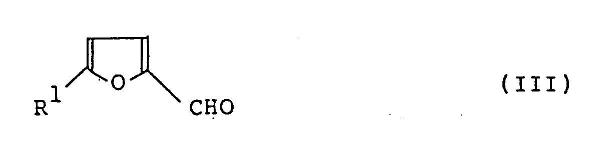 worin R₁ für ein Wasserstoffatom oder eine Methylgruppe steht, mit einer organischen Dihalogenidverbindung der Formel (IV), 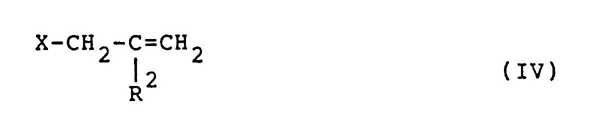 worin R₂ und X jeweils unabhängig voneinander für ein Chlor-, Brom- oder Jodatom stehen, in Gegenwart von Zink in einem Lösungsmittel, ausgewählt aus Wasser und einem Gemisch aus Wasser und einem organischen Lösungsmittel, hergestellt wurde.

14. Verfahren zur Herstellung von Furylpropargylcarbinol oder eines Derivats desselben nach Anspruch 13, wobei das Gemisch aus Wasser und einem organischen Lösungsmittel Wasser als Hauptkomponente enthält.

15. Verfahren zur Herstellung von Furylpropargylcarbinol oder eines Derivats desselben nach Anspruch 13, wobei die Umsetzung von Furfural oder eines Derivats desselben mit einer organischen Dihalogenidverbindung in Gegenwart einer Säure durchgeführt wird.

16. Verfahren zur Herstellung von Furylpropargylcarbinol oder eines Derivats desselben nach Anspruch 14, wobei die Umsetzung von Furfural oder eines Derivats desselben mit einer organischen Dihalogenidverbindung in Gegenwart einer Säure durchgeführt wird.

17. Verfahren zur Herstellung von Furylpropargylcarbinol oder eines Derivats desselben nach Anspruch 15, wobei die Säure aus der Gruppe Essigsäure, Chlorwaserstoffsäure und Schwefelsäure ausgewählt wird.

18. Verfahren zur Herstellung von Furylpropargylcarbinol oder eines Derivats desselben nach Anspruch 13, wobei die organische Dihalogenidverbindung aus 2,3-Dichlor-1-propen besteht.

19. Verfahren zur Herstellung von Furylpropargylcarbinol oder eines Derivats desselben nach Anspruch 14, wobei die organische Dihalogenidverbindung aus 2,3-Dichlor-1-propen besteht.

20. Verfahren zur Herstellung von Furylpropargylcarbinol oder eines Derivats desselben nach Anspruch 15, wobei die organische Dihalogenidverbindung aus 2,3-Dichlor-1-propen besteht.

21. Verfahren zur Herstellung von Furylpropargylcarbinol oder eines Derivats desselben nach Anspruch 16, wobei die organische Dihalogenidverbindung aus 2,3-Dichlor-1-propen besteht.

22. Verfahren zur Herstellung von Furylpropargylcarbinol oder eines Derivats desselben nach Anspruch 17, wobei die organische Dihalogenidverbindung aus 2,3-Dichlor-1-propen besteht.

23. Verfahren zur Herstellung von Furylpropargylcarbinol oder eines Derivats desselben nach Anspruch 13, wobei das Halogenallylfurancarbinol aus 2'-Chlorallyl-5-methylfurylcarbinol oder 2-Chlorallylfurylcarbinol besteht.

24. Verfahren zur Herstellung von Furylpropargylcarbinol oder eines Derivats desselben nach Anspruch 13, wobei die organische Dihalogenidverbindung aus 2,3-Dibrom-1-propen besteht.

25. Verfahren zur Herstellung von Furylpropargylcarbinol oder eines Derivats desselben nach einem der Ansprüche 18 und 24, wobei die Base aus einem Alkalimetallhydroxid oder einem Erdalkalimetallhydroxid besteht.

26. Verfahren zur Herstellung von Furylpropargylcarbinol oder eines Derivats desselben nach Anspruch 25, wobei bei dem Verfahren zur Umsetzung von Furfural oder eines Derivats desselben der Formel (III) mit einer organischen Dihalogenidverbindung der Formel (IV) das Lösungsmittel aus einem Gemisch aus Wasser und einem polaren aprotischen Lösungsmittel besteht.

27. Verfahren zur Herstellung von Furylpropargylcarbinol oder eines Derivats desselben nach Anspruch 25, wobei bei dem Verfahren zur Umsetzung von Furfural oder eines Derivats desselben der Formel (III) mit einer organischen Dihalogenidverbindung der Formel (IV) das Lösungsmittel aus Wasser besteht.

28. Verfahren zur Herstellung von Furylpropargylcarbinol oder eines Derivats desselben nach Anspruch 25, wobei das Lösungsmittel aus einem Gemisch aus Wasser und irgendeinem hydrophoben Kohlenwasserstoff oder hydrophoben halogenierten Kohlenwaserstoff besteht.

29. Halogenallylfurancarbinol oder ein Derivat desselben der Formel (II), 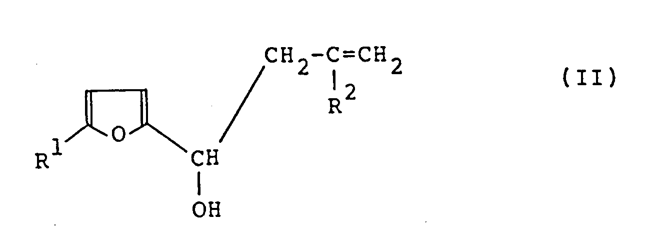 worin bedeuten:
R₁ ein Wasserstoffatom oder eine Methylgruppe und
R₂ ein Chlor-, Brom- oder Jodatom.

30. Halogenallylfurancarbinol oder ein Derivat desselben nach Anspruch 29, wobei R₁ für ein Wasserstoffatom steht und R₂ ein Chloratom darstellt.

31. Halogenallylfurancarbinol oder ein Derivat desselben nach Anspruch 29, wobei R₁ für eine Methylgruppe steht und R₂ ein Chloratom darstellt.

32. Halogenallylfurancarbinol oder ein Derivat desselben nach Anspruch 29, wobei R₁ für ein Wasserstoffatom steht und R₂ ein Bromatom darstellt.

33. Halogenallylfurancarbinol oder ein Derivat desselben nach Anspruch 29, wobei R₁ für eine Methylgruppe steht und R₂ ein Bromatom darstellt.

34. Verfahren zur Herstellung eines Halogenallylfurancarbinols oder eines Derivats desselben der Formel (II), 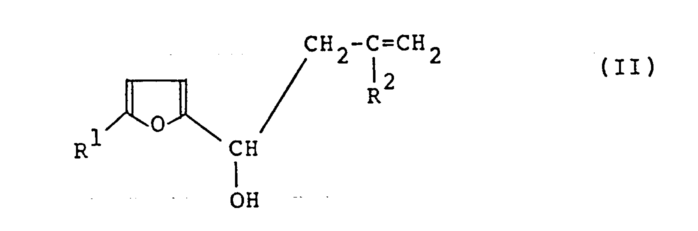 worin bedeuten:
R₁ ein Wasserstoffatom oder eine Methylgruppe und
R₂ ein Chlor-, Brom- oder Jodatom,
durch Umsetzen von Furfural oder eines Derivats desselben der Formel (III), 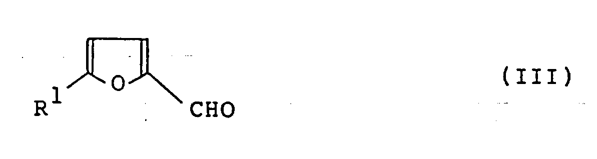 worin R₁ für ein Wasserstoffatom oder eine Methylgruppe steht,
mit einer organischen Dihalogenidverbindung der Formel (IV), 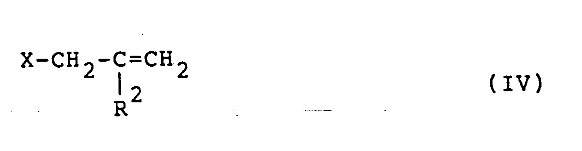 worin R₂ und X jeweils unabhängig voneinander für ein Chlor-, Brom- oder Jodatom stehen, in Gegenwart von Zink in einem Lösungsmittel, ausgewählt aus Wasser und einem Gemisch aus Wasser und einem organischen Lösungsmittel.

35. Verfahren zur Herstellung eines Halogenallylfurancarbinols oder eines Derivats desselben nach Anspruch 34, wobei die organische Dihalogenidverbindung aus 2,3-Dichlor-1-propen oder 2,3-Dibrom-1-propen besteht.

36. Verfahren zur Herstellung eines Halogenallylfurancarbinols oder eines Derivats desselben nach Anspruch 34, wobei die Umsetzung in Gegenwart einer Säure durchgeführt wird.

37. Verfahren zur Herstellung eines Halogenallylfurancarbinols oder eines Derivats desselben nach Anspruch 36, wobei die organische Dihalogenidverbindung aus 2,3-Dichlor-1-propen oder 2,3-Dibrom-1-propen besteht.

## Revendications

1. Procédé de production du furylpropargylcarbinol ou d'un de ses dérivés représentés par la formule (I) : 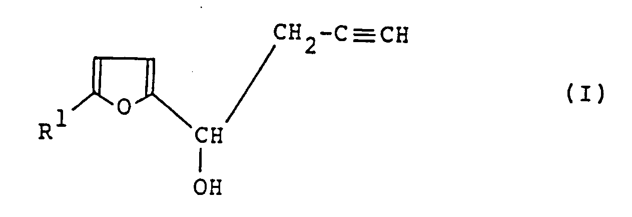 dans laquelle R¹ représente un atome d'hydrogène ou un groupement méthyle, procédé qui constate à soumettre un haloallylfurannecarbinol ou un de ses dérivés représentés par la formule (II) : 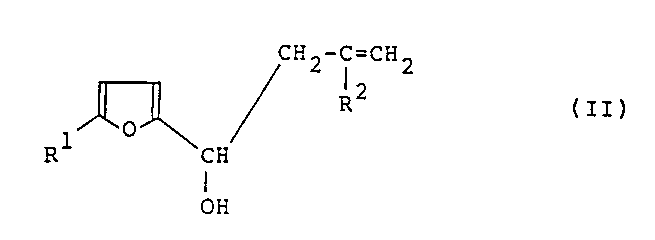 dans laquelle R¹ représente un atome d'hydrogène ou un groupement méthyle et R² représente un atome de chlore, un atome de brome ou un atome d'iode, à une réaction de déshydrobalogénation avec une base dans un solvant de réaction.

2. Procédé de production du furylpropargylcarbinol ou d'un de ses dérivés selon la revendication 1, dans lequel la base est un hydroxyde de métal alcalin ou un hydroxyde de métal alcalino-terreux.

3. Procédé de production du furylpropargylcarbinol ou d'un de ses dérivés selon la revendication 1, dans lequel le solvant de réaction est un mélange d'eau et d'un quelconque hydrocarbure hydrophobe ou hydrocarbure halogéné hydrophobe.

4. Procédé de production du furylpropargylcarbinol ou d'un de ses dérivés selon la revendication 1, dans lequel le solvant de réaction est l'eau.

5. Procédé de production du furylpropargylcarbinol ou d'un de ses dérivés selon la revendication 1, dans lequel le solvant de réaction est un solvant aprotique polaire.

6. Procédé de production du furylpropargylcarbinol ou d'un de ses dérivés selon la revendication 2, dans lequel le solvant de réaction est un mélange d'eau et d'un quelconque hydrocarbure hydrophobe ou hydrocarbure halogéné hydrophobe.

7. Procédé de production du furylpropargylcarbinol ou d'un de ses dérivés selon la revendication 2, dans lequel le solvant de réaction est l'eau.

8. Procédé de production du furylpropargylcarbinol ou d'un de ses dérivés selon la revendication 2, dans lequel le solvant de réaction est un solvant aprotique polaire.

9. Procédé de production du furylpropargylcarbinol ou d'un de ses dérivés selon la revendication 1, dans lequel la réaction de déshydrohalogénation est effectuée en présence d'un catalyseur de transfert de phase.

10. Procédé de production du furylpropargylcarbinol ou d'un de ses dérivés selon la revendication 9, dans lequel le solvant de réaction est l'eau.

11. Procédé de production du furylpropargylcarbinol ou d'un de ses dérivés selon la revendication 9, dans lequel le solvant de réaction est un mélange d'eau et d'un quelconque hydrocarbure hydrophobe ou hydrocarbure halogéné hydrophobe.

12. Procédé de production du furylpropargylcarbinol ou d'un de ses dérivés selon la revendication 2, dans lequel la réaction de déshydrohalogénation est effectuée en présence d'un catalyseur de transfert de phase.

13. Procédé de production du furylpropargylcarbinol ou d'un de ses dérivés selon la revendication 1, dans lequel l'haloallylfurannecarbinol ou son dérivé sont obtenus selon un procédé qui consiste à faire réagir du furfural ou un de ses dérivés représentés par la formule (III) : 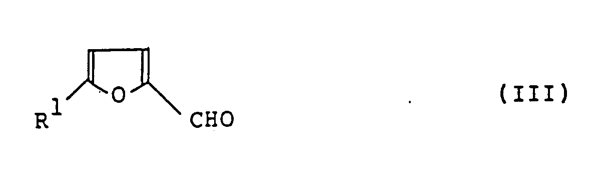 dans laquelle R¹ représente un atome d'hydrogène ou un groupement méthyle, avec un dihalogénure organique représenté par la formule (IV) : 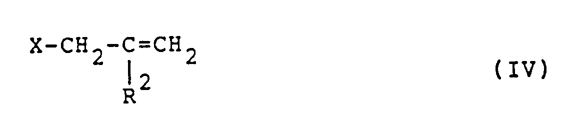 dans laquelle chacun de R² et X représente indépendamment un atome de chlore, un atome de brome ou un atome d'iode,
en présence de zinc dans un solvant choisi entre l'eau et un mélange d'eau et de solvant organique.

14. Procédé de production du furylpropargylcarbinol ou d'un de ses dérivés selon la revendication 13, dans lequel le mélange d'eau et de solvant organique contient de l'eau comme composant principal.

15. Procédé de production du furylpropargylcarbinol ou d'un de ses dérivés selon la revendication 13, dans lequel la réaction du furfural ou d'un de ses dérivés avec un dihalogénure organique est effectuée en présence d'un acide.

16. Procédé de production du furylpropargylcarbinol ou d'un de ses dérivés selon la revendication 14, dans lequel la réaction du furfural ou d'un de ses dérivés avec un dihalogénure organique est effectuée en présence d'un acide.

17. Procédé de production du furylpropargylcarbinol ou d'un de ses dérivés selon la revendication 15, dans lequel l'acide est choisi dans le groupe comprenant l'acide acétique, l'acide chlorhydrique et l'acide sulfurique.

18. Procédé de production du furylpropargylcarbinol ou d'un de ses dérivés selon la revendication 13, dans lequel le dihalogénure organique est le 2,3-dichloro-1-propène.

19. Procédé de production du furylpropargylcarbinol ou d'un de ses dérivés selon la revendication 14, dans lequel le dihalogénure organique est le 2,3-dichloro-1-propène.

20. Procédé de production du furylpropargylcarbinol ou d'un de ses dérivés selon la revendication 15, dans lequel le dihalogénure organique est le 2,3-dichloro-1-propène.

21. Procédé de production du furylpropargylcarbinol ou d'un de ses dérivés selon la revendication 16, dans lequel le dihalogénure organique est le 2,3-dichloro-1-propène.

22. Procédé de production du furylpropargylcarbinol ou d'un de ses dérivés selon la revendication 17, dans lequel le dihalogénure organique est le 2,3-dichloro-1-propène.

23. Procédé de production du furylpropargylcarbinol ou d'un de ses dérivés selon la revendication 13, dans lequel l'haloallylfurannecarbinol est le 2'-chloroallyl-5-méthylfurylcarbinol ou le 2-chloroallylfurylcarbinol.

24. Procédé de production du furylpropargylcarbinol ou d'un de ses dérivés selon la revendication 13, dans lequel le dihalogénure organique est le 2,3-dibromo-1-propène.

25. Procédé de production du furylpropargylcarbinol ou d'un de ses dérivés selon l'une ou l'autre des revendications 18 et 24, dans lequel la base est un hydroxyde de métal alcalin ou un hydroxyde de métal alcalino-terreux.

26. Procédé de production du furylpropargylcarbinol ou d'un de ses dérivés selon la revendication 25, dans lequel, dans le procédé qui consiste à faire réagir le furfural ou un de ses dérivés représentés par la formule (III) avec un dihalogénure organique représenté par la formule (IV), le solvant est un mélange d'eau et de solvant aprotique polaire.

27. Procédé de production du furylpropargylcarbinol ou d'un de ses dérivés selon la revendication 25, dans lequel, dans le procédé qui consiste à faire réagir le furfural ou un de ses dérivés représentés par la formule (III) avec un dihalogénure organique représenté par la formule (IV), le solvant est l'eau.

28. Procédé de production du furylpropargylcarbinol ou d'un de ses dérivés selon la revendication 25, dans lequel le solvant est un mélange d'eau et d'un quelconque hydrocarbure hydrophobe ou hydrocarbure halogéné hydrophobe.

29. Haloallylfurannecarbinol ou un de ses dérivés représentés par la formule (II) : 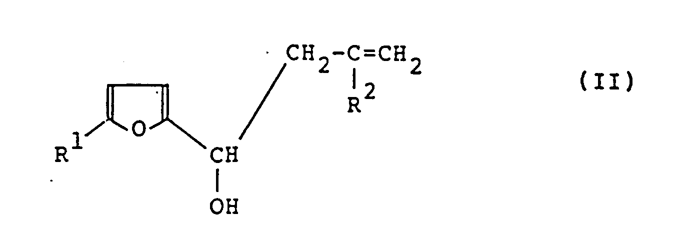 dans laquelle R¹ représente un atome d'hydrogène ou un groupement méthyle et R² représente un atome de chlore, un atome de brome ou un atome d'iode.

30. Haloallylfurannecarbinol ou un de ses dérivés selon la revendication 29, dans lequel R¹ est un atome d'hydrogène et R² est un atome de chlore.

31. Haloallylfurannecarbinol ou un de ses dérivés selon la revendication 29, dans lequel R¹ est un groupement méthyle et R² est un atome de chlore.

32. Haloallylfurannecarbinol ou un de ses dérivés selon la revendication 29, dans lequel R¹ est un atome d'hydrogène et R² est un atome de brome.

33. Haloallylfurannecarbinol ou un de ses dérivés selon la revendication 29, dans lequel R¹ est un groupement méthyle et R² est un atome de brome.

34. Procédé de production d'un haloallylfurannecarbinol ou d'un de ses dérivés représentés par la formule (II) : 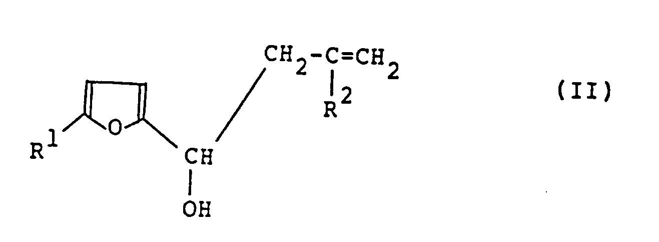 dans laquelle R¹ représente un atome d'hydrogène ou un groupement méthyle et R² représente un atome de chlore, un atome de brome ou un atome d'iode,
qui consiste à faire réagir du furfural ou un de ses dérivés représentés par la formule (III) : 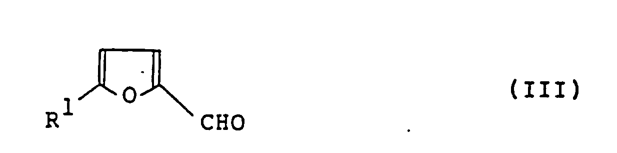 dans laquelle R¹ représente un atome d'hydrogène ou un groupement méthyle,
avec un dihalogénure organique représenté par la formule (IV) : 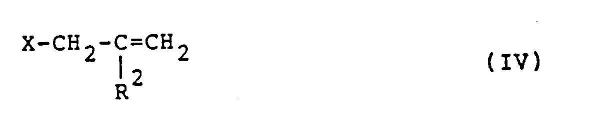 dans laquelle chacun de R² et X représente indépendamment un atome de chlore, un atome de brome ou un atome d'iode, en présence de zinc dans un solvant choisi entre l'eau et un mélange d'eau et de solvant organique.

35. Procédé de production d'un haloallylfurannecarbinol ou d'un de ses dérivés selon la revendication 34, dans lequel le dihalogénure organique est le 2,3-dichloro-1-propène ou le 2,3-dibromo-1-propène.

36. Procédé de production d'un haloallylfurannecarbinol ou d'un de ses dérivés selon la revendication 34, dans lequel la réaction est effectuée en présence d'un acide.

37. Procédé de production d'un haloallylfurannecarbinol ou d'un de ses dérivés selon la revendication 36, dans lequel le dihalogénure organique est le 2,3-dichloro-1-propène ou le 2,3-dibromo-1-propène.
